# EUROPEAN PATENT SPECIFICATION

(11) **EP 3 137 069 B1**
(45) Date of publication and mention of the grant of the patent: **14.03.2018**
(21) Application number: 14777935.9
(22) Date of filing: 04.07.2014
(51) Int. Cl.: A61K 31/192, A61K 9/16, B82Y 5/00, A61P 35/00

(54) **NANOPARTICLE FORMULATION CONTAINING FARNESYLTHIOSALICYLIC ACID (SALIRASIB)**
NANOPARTIKEL-ZUSAMMENSETZUNG ENTHALTEND FARNESYLTHIOSALICYLSÄURE (SALIRASIB)
FORMULATION NANOPARTICULAIRE COMPRENANT DE L'ACIDE FARNESYLTHIOSALICILIQUE (SALIRASIB)

(30) Priority: 28.04.2014 TR 201404753
(43) Date of publication of application: 08.03.2017
(73) Proprietor: Pehlivan, Sibel, Ankara (TR); Vural, Imran, Ankara (TR); Kaffashi, Abbas, Ankara (TR)
(72) Inventor: LÜLE, Sevda, Ankara (TR); KOSUCU, Hüsnü, Ankara (TR); ASKUN, Melike Mut, Ankara (TR); DEMIR, Taner, Ankara (TR); SARISÖZEN, Can, Ankara (TR); BUGDAYCI, Emre, Burdur (TR); SÖYLEMEZOGLU, Figen, Ankara (TR); OGUZ, Hatice Kader Karli, Ankara (TR)
(74) Representative: Bulut, Pinar
(86) International application number: PCT/TR2014/000248
(87) International publication number: WO 2015/167408

(56) References cited:
- XIAOLAN ZHANG ET AL: "PEG-Farnesylthiosalicylate Conjugate as a Nanomicellar Carrier for Delivery of Paclitaxel", BIOCONJUGATE CHEMISTRY, vol. 24, no. 3, 20 March 2013 (2013-03-20) , pages 464-472, XP055148284, ISSN: 1043-1802, DOI: 10.1021/bc300608h
- KRAITZER A ET AL: "Novel farnesylthiosalicylate (FTS)-eluting composite structures", EUROPEAN JOURNAL OF PHARMACEUTICAL SCIENCES, ELSEVIER, AMSTERDAM, NL, vol. 37, no. 3-4, 28 June 2009 (2009-06-28), pages 351-362, XP026160264, ISSN: 0928-0987, DOI: 10.1016/J.EJPS.2009.03.004 [retrieved on 2009-03-21]
- LIANGFANG ZHANG ET AL: "Self-Assembled Lipid - Polymer Hybrid Nanoparticles: A Robust Drug Delivery Platform", ACS NANO, AMERICAN CHEMICAL SOCIETY, US, vol. 2, no. 8, 1 January 2008 (2008-01-01) , pages 1696-1702, XP009142473, ISSN: 1936-0851
- ZHONG QIU ET AL: "Optimization of DNA delivery by three classes of hybrid nanoparticle/DNA complexes", JOURNAL OF NANOBIOTECHNOLOGY, BIOMED CENTRAL, GB, vol. 8, no. 1, 24 February 2010 (2010-02-24), page 6, XP021068102, ISSN: 1477-3155

## Description

### Technical Part

The purpose of targeted chemotherapy is to change the disposition, uptake or efficacy of antineoplastic drugs by providing more damages to tumor cells rather than normal cells. Since the tumor locate in brain, in addition to targeting, blood brain barrier (BBB) has to be passed. It is known that succesfull applications are carried out with nanotechnology based drug delivery systems such as liposomes, nanoparticles, dendrimers and micelles in order to pass BBB and to decrease side effects in healty tissues.

Farnesylthiosalicylic acid (Salirasib, FTA) (Figure 1), a new generation antineoplastic agent, is a specific inhibitor of Ras protein, which presents in high rate in most of malignant tumors. FTA is insoluble in water, soluble (20 mg/mL) in organic solvents like ethanol, DMSO and dimethylformamide. Recently, it has been found that FTA is effective against brain tumors in in vitro cell culture studies. However, FTA can not croos the BBB in effective doses.

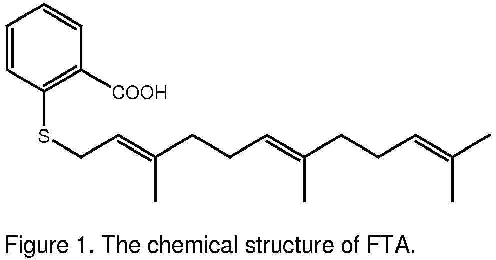

Xialon discloses the nanomicellar formulations containing Paclitaxel. In this study, the formulations contain polyethylene glycol (PEG) conjugated farnesyl thiosalicylic acid as an excipient. (XIAOLAN ZHANG ET AL, "PEG-Farnesylthiosalicylate Conjugate as a Nanomicellar Carrier for Delivery of Paclitaxel", BIOCONJUGATE CHEMISTRY, (2013), vol. 24, no. 3, pages 464 - 472.

Kraitzer discloses ,the Farnesylthiosalicylate (FTS)-eluting core/shell composite fiber formulations in order to apply as drug-eluting stents and the incorporation of FTS in a stent coating. The formulations were composed of a polyglyconate core and a porous poly(d,l-lactic-glycolic acid) shell loaded with FTS, prepared using freeze drying of inverted emulsions. KRAITZER A ET AL, "Novel farnesylthiosalicylate (FTS)-eluting composite structures", EUROPEAN JOURNAL OF PHARMACEUTICAL SCIENCES, ELSEVIER, AMSTERDAM, NL, vol. 37, no. 3-4, (2009), pages 351 - 362

### Description of Invention

This invention is related to FTA loaded PLGA nanoparticles functionalized with lipid/cationic lipid-PEG (hybrid), which can cross the BBB in effective concentration. Formulations can be administered intratumorally or intravenously. The cationic properties of the hybrid nanoparticles and intratumoral route of administration have increased the succes of tumor treatment.

Hybrid nanoparticle systems prepared are formed by a hydrophobic polymeric core and a hydrophilic polymeric shell separated by a lipid monolayer. Lipid-polymer hybrid nanoparticles are drug delivery systems which integrate the advantages of both liposomes and polymeric nanoparticles (while liposomes easily interact with biological membranes, nanoparticles are very stable sytems). Furthermore, they have some additional advantages such as providing high encapsulation ratio for water insoluble drugs, sustained drug release, excellent serum stability and targeting to different cells.

Poly(lactic-co-glycolic) acid (PLGA), a synthetic biodegradable, biocompatible, non-toxic and FDA approved polymer, was selected as a core (relatively hydrophobic polymer) for the preparation of hybrid nanoparticles. PLGA (copolymer ratio 50:50 or 85:15), as a polymer, 1,2 distearoyl-glycerol-3-phospho-ethanolamine-N [methoxy (PEG)-2000] ammonium salt containing PEG (mw 2000 Da) (DSPE-PEG),as a lipid-PEG conjugate, are used in the formulations. PLGA-DSPE-PEG hybrid nanoparticles are prepared by emulsion sonication method.

1,2-dioleyl-3-trimetilammonium-propane (DOTAP) was used in formulations in order to obtain positive charge.

The formulation comprising salirasib characterized in that it comprises PLGA-DSPE-PEG-DOTAP hybrid nanoparticles.

Optimum particle size was achieved by preformulation studies. Then the formulation ratios providing optimum particle size was used to prepare drug loaded formulations.

Some of the chemicals were purchased from following companies:

| | |
|---|---|
| Farnesylthiosalicylic acid (FTA) | Concordia Pharmaceuticals Inc, USA |
| PLGA (50:50) (Resomer RG 503) | Boehringer Ingelheim, Germany |
| PLGA (85:15) (Resomer RG 753 S) | Boehringer Ingelheim, Germany |
| 1,2-dioleyl-3-trimetilammonium-propane (DOTAP) | Avanti, USA |
| 1,2 distearoyl-glycerol-3-phospho-ethanolamine-N [methoxy (PEG)-2000] ammonium salt (DSPE-PEG) | Avanti, USA |
| Lecithin (from egg yolk, Type XVI-E, ≥99% (TLC), Liyophilized powder | Avanti, USA |

Nanoparticles containing salirasib were obtained following process:
   a) Organic phase (OP)containing PLGA is prepared,
   b) FTA is added to the organic phase,
   c) Lecithin, DSPE-PEG are separately dissolved in 4% (v/v) ethanol preferentially, and then mixed in suitable amount, proper amount of deionized water is added to this mixing,
   d) Aqeous phase (AP) is added to organic phase preferentially in 10:1 ratio,
   e) AP and OP mixing is sonicated for 5 min using a bath sonicator at a frequency of 42 kHz and power of 100 W,
   f) The formed nanoparticles are centrifuged in filter-centrifuge tubes containing a 10 kDa molecular size permeable membrane (Millipore),
   g) The nanoparticles are washed three times using PBS (pH 7.4),
   h) The nanoparticle dispersions are frozen at -80°C and lyophilized for 24 hours.

For the preparation of hybrid nanoparticles containing DOTAP step c is carried out as follows:
c) Lecithin, DSPE-PEG and DOTAP are separately dissolved in 4% (v/v) ethanol preferentially, and then mixed in suitable amount, proper amount of deionized water is added to this mixing.

### Detailed Description of the Invention

### Preparation of Hybrid Nanoparticles

For the preparation of PLGA-DSPE-PEG hybrid nanoparticles, PLGA was dissolved in acetonitrile (2.5 µg/mL) to obtain organic phase. FTA (5%, 10%, and 20% (w/w) of polymer amount) was added into organic phase. To obtain aqueous phase, lecithin and DSPE-PEG (and DOTAP for DOTAP containing particles) were separately dissolved in 4% (v/v) ethanol, and then certain amount of these solutions mixed with deionized water. This aqueous phase was added into organic phase (ratio, 10:1) and sonicated in a capped glass vial for 5 min using a bath sonicator at a frequency of 42 kHz and power of 100 W. The formed nanoparticles were centrifuged in filter-centrifuge tubes containing a 10 kDa molecular size permeable membrane (Millipore, 10 kDa), and was washed three times using PBS (pH 7.4). Nanoparticle dispersions obtained were frozen at -80°C and lyophilized for 24 hours.

### Preparation of Hybrid Nanoparticles Containing DOTAP

1,2-dioleyl-3-trimetilammonium-propane (DOTAP) is a positively charged (monocationic) lipid. It provides both of efficient transport of DOTAP containing formulations into the cell and complexing with DNA. Furthermore, positively charged nanoparticles lead to succesfull results to cross BBB.

The formulation ratios providing optimum particle size for DOTAP free nanoparticles was used to prepare drug loaded formulations with DOTAP.

For the preparation of hybrid nanoparticles with DOTAP, PLGA was dissolved in acetonitrile (2.5 µg/mL) to obtain organic phase. FTA (5%, 10%, and 20% (w/w) of polymer amount) was added into organic phase. To obtain aqueous phase, lecithin, DSPE-PEG and DOTAP were separately dissolved in 4% (v/v) ethanol, and then certain amount of these solutions mixed with deionized water. This aqueous phase was added into organic phase (ratio, 10:1) and sonicated in a capped glass vial for 5 min using a bath sonicator at a frequency of 42 kHz and power of 100 W. The formed nanoparticles were centrifuged in filter-centrifuge tubes containing a 10 kDa molecular size permeable membrane (Millipore, 10 kDa), and was washed three times using PBS (pH 7.4). Nanoparticle dispersions obtained were frozen at -80°C and lyophilized for 24 hours.

**Table 1. Formulation of hybrid nanoparticles containing DOTAP**

| Formulation | PLGA (µmol) | Lecithin (µmol) | DOTAP (µmol) | PEG-PE (µmol) |
|---|---|---|---|---|
| PEG-PE | 0,015 | 0,017 | - | 0,07 |
| PEG-PE/DOTAP(50:50) | 0,015 | 0,017 | 0,143 | 0,035 |
| PEG-PE/DOTAP(75:25) | 0,015 | 0,017 | 0,072 | 0,053 |

### Particle Size

It is determined that the particle size is below 200 nm for the nanoparticle systems in order to treat the brain tumors (Table 2).

**Table 2. The particle size of prepared hybrid nanoparticle formulations**

| Formulation | Particle size (nm) (Avarage±Standard Deviation) |
|---|---|
| 50:50 PLGA/DSPE-PEG (Blank) | 94 ± 2,3 |
| 85:15 PLGA/DSPE-PEG (Blank) | 102 ± 2,0 |
| 50:50 PLGA/ DSPE-PEG (%5 FTA loaded) | 121 ± 3,2 |
| 85:15 PLGA/DSPE-PEG(%5 FTA laoded | 147 ± 0,7 |
| 50:50 PLGA/ DSPE-PEG with DOTAP (Blank) | 120 ± 3,4 |
| 85:15 PLGA/ DSPE-PEG with DOTAP (Blank) | 145 ± 2,5 |
| 50:50 PLGA/ DSPE-PEG with DOTAP (%5 FTA loaded) | 127 ± 2,0 |
| 85:15 PLGA/ DSPE-PEG with DOTAP (%5 FTA laoded) | 155 ± 2,7 |

### FTA Loading

FTA can be loaded to the nanoparticles between 1-30 % of polymer amount (w/w). 5,10 or 20% of poymer amount of FTA was loaded to the nanoparticles. Since higher drug amount (10 and 20% (w/w) of polymer amount) did not provide higher drug loading efficiency in the formulations, 5% of polymer amount of FTA was selected as target drug loading.

The formulations without DOTAP, drug loading efficiencies were determined as 21±2% and 28±1.2 %, for formulations containing PLGA 50:50 and 85:15 copolymer,respectively.

The highest drug loading value was obtained by the formulation in which PLGA 50:50 copolymer ratio was used, and DSPE-PEG/DOTAP ratio was 75:25.

### Results

RG2 cells, which is mentioned as one of the most similar cell groups to human glioma cells, were used as brain tumor cell line.

The formulations in which PLGA 50:50 copolymer ratio was used were selected to evaluate in cell culture studies.

DOTAP containing nanoparticles showed the highest anti-cancer activity in cell culture studies.

It was found that FTA-loaded PLGA(50:50)-DSPE-PEG-DOTAP hybrid nanoparticles were active against glioblastoma following the intratumoral and intravenous route of administration to the rats.

To determine the presence and size of tumor, magnetic resonance (MR) imaging was performed during in vivo studies. Also, histological evaluation were carried out to assign the size of the tumor.

Following the intravenous therapy, comparing to initial value, tumor size increased by 106%, 63.47 % and 130.02 in control, drug solution and blank nanoparticle group, respectively. Tumor size decreased by 76.20 % in FTA loaded nanoparticle group.

After the intratumoral therapy, comparing to initial value, tumor size increased by 106%, 59 % and 74.72 in control, drug solution and blank nanoparticle group, respectively. Tumor size decreased by 88.62 % in FTA loaded nanoparticle group.

It was seen that tumor decrease ratio obtained with intratumoral application was significantly higher than with intravenous application (88.62% vs. 76.20%).

**Table 3. MR imaging defined pre- and post-treatment tumor size of in vivo study groups that received intravenous injection (n=3 for each group, Average±SD)**

| Formulation | Pre treatment Tumor Size (mm²) | Post treatment tumor size (mm²) | Change in tumor size (%) |
|---|---|---|---|
| Drug Solution | 23.49 ± 5.7 | 38.4 ± 3.2 | **(+)** 63,47 ± 1,1 |
| Blank Nanoparticle Suspension | 21.18 ± 7.17 | 48.72 ± 7.3 | **(+)** 130,02 ± 1,0 |
| FTA Loaded Nanoparticle Suspension | 22.65 ± 6.43 | 5.39 ± 5.8 | **(-)** 76,20 ± 1,2 |
| Control group (no treatment) | 23.99 ± 5.3 | 49.56 ± 6.33 | **(+)** 106 ± 1,4 |

**Table 4. MR imaging defined pre- and post-treatment tumor size of in vivo study groups that received intratumoral injection (n=3 for each group, Avarage±SD)**

| Formulation | Pre treatment Tumor Size (mm²) | Post treatment tumor size (mm²) | Change in tumor size (%) |
|---|---|---|---|
| Drug Solution | 19.71 ± 2.4 | 31.4 ± 1.35 | **(+)** 59 ± 1,3 |
| Blank Nanoparticle Suspension | 24.45 ± 1.76 | 42.72 ± 2.3 | **(+)** 74,72 ± 1,2 |
| FTA Loaded Nanoparticle Suspensiom | 22.15 ± 2.23 | 2.52 ± 1.33 | **(-)** 88,62 ± 1,2 |
| Control group (no treatment) | 23.99 ± 1.3 | 49.56 ± 2.13 | **(+)** 106,58 ± 1,4 |

## Claims

1. The formulation comprising salirasib **characterized in that** it comprises PLGA-DSPE-PEG-DOTAP hybrid nanoparticles.

2. The formulation comprising salirasib according to claim 1, wherein it comprises PLGA 50:50 and/or PLGA 85:15 copolymers.

3. The formulation comprising salirasib according to claim 1, wherein it comprises PLGA 50:50 copolymer.

4. The formulation comprising salirasib according to claim 1, wherein it comprises 1,2 distearoyl-glycerol-3-phospho-ethanolamine-N [methoxy (PEG)-2000] ammonium salt (DSPE-PEG) as a lipid-PEG conjugate.

5. The formulation comprising salirasib according to claim 1, wherein PEG chain has a molecular weight of 2000 Da.

6. The formulation comprising salirasib according to claim 1, wherein FTA amount is between 1%-30% of polymer amount in weight (w/w).

7. The formulation comprising salirasib according to claim 1, wherein FTA amount is between 5%-20% of polymer amount in weight (w/w).

8. The formulation comprising salirasib according to claim 1, wherein FTA amount is 5 % of polymer amount in weight (w/w).

9. The formulation comprising salirasib according to claim 1, wherein it is obtained by the process below:
a) Organic phase (OP) containing PLGA is prepared,
b) FTA is added to the organic phase,
c) Lecithin, DSPE-PEG and DOTAP are separately dissolved in 4% (v/v) ethanol preferentially, and then mixed in suitable amount, proper amount of deionized water is added to this mixing,
d) Aqueous phase (AP) is added to organic phase preferentially in 10:1 ratio,
e) OP and AP mixing is sonicated for 5 min using a bath sonicator at a frequency of 42 kHz and power of 100 W,
f) The formed nanoparticles are centrifuged in filter-centrifuge tubes containing a 10 kDa molecular size permeable membrane (Millipore),
g) The nanoparticles are washed three times using PBS (pH 7.4),
h) The nanoparticle dispersions are frozen at -80°C and lyophilized for 24 hours.

10. The formulation comprising salirasib according to claim 1, wherein it is administered intravenously.

11. The formulation comprising salirasib according to claim 1, wherein it comprises it is administered intratumorally.

12. The formulation comprising salirasib according to claim 1 for use in the treatment of brain tumors.

## Patentansprüche

1. Salirasib enthaltende Formulierung **gekennzeichnet durch** die Implikation der PLGA-DSPE-PEG-DOTAP-Hybrid-Nanopartikeln.

2. Salirasib enthaltende Formulierung nach Anspruch 1, die die PLGA 50:50 und / oder PLGA 85:15 Copolymere umfasst.

3. Salirasib enthaltende Formulierung nach Anspruch 1, die die PLGA 50:50 Copolymere umfasst.

4. Salirasib enthaltende Formulierung nach Anspruch 1, die das1,2-Distearoylglycerol-3-phosphoethanolamin-N [methoxy (PEG) -2000] Ammoniumsalz als (DSPE-PEG) eine Lipid-PEG-Konjugation umfasst.

5. Salirasib enthaltende Formulierung nach Anspruch 1, die das 2000 Da Molekulargewicht der PEG Kette aufweist.

6. Salirasib enthaltende Formulierung nach Anspruch 1, bei der die FTA Gewichtsmenge (w/w) in einem Bereich von 1 % bis 30 % der Polimermenge liegt.

7. Salirasib enthaltende Formulierung nach Anspruch 1, bei der die FTA Gewichtsmenge (w/w) in einem Bereich von 5 % bis 20 % der Polimermenge liegt.

8. Salirasib enthaltende Formulierung nach Anspruch 1, bei der die FTA Gewichtsmenge (w/w) 5 % der Polimermenge liegt.

9. Salirasib enthaltende Formulierung nach Anspruch 1, die durch den folgenden Prozess erhalten wird:
a) Organische Phase (OP), die PLGA enthält, wird hergestellt,
b) FTA wird der organischen Phase zugesetzt,
c) Lecithin, DSPE-PEG und DOTAP werden vorzugsweise getrennt in 4% (Volumen/ Volumen) Ethanol gelöst und dann in einer geeigneten Menge gemischt, wobei eine geeignete Menge deionisiertes Wasser zu diesem Mischen hinzugefügt wird.
d) Wässrige Phase (AP) wird vorzugsweise im Verhältnis 10: 1 zur organischen Phase gegeben,
e) Die OP- und AP-Mischung wird für 5 min mit einem Ultraschallbad mit einer Frequenz von 42 kHz und einer Leistung von 100 W beschallt,
f) Die erhaltenen Nanopartikeln werden in Filterzentrifugenröhrchen zentrifugiert, die eine durchlässige Membran mit einer molekularen Größe von 10 kDa (Millipore) enthalten.
g) Die Nanopartikeln werden 3 mal mit PBS (pH 7,4) gewaschen
h) Die Nanopartikeldispersionen werden bei -80°C eingefroren und für 24 Stunden lyophilisiert.

10. Salicasib enthaltende Formulierung nach Anspruch 1, die intravenös verabreicht wird.

11. Salicasib enthaltende Formulierung nach Anspruch 1, die als Intra-Tumor verabreicht wird.

12. Salicasib enthaltende Formulierung nach Anspruch 1, die bei der Behandlung der Gehirntumoren benutzt wird.

## Revendications

1. Formulation comprenant du salirasib **caractérisée en ce qu'**elle comprend des nanoparticules hybrides PLGA-DSPE-PEG-DOTAP.

2. Formulation comprenant du salirasib selon la revendication 1, dans laquelle elle comprend des copolymères PLGA 50:50 et / ou PLGA 85:15.

3. Formulation comprenant du salirasib selon la revendication 1, dans laquelle elle comprend un copolymère PLGA 50:50.

4. Formulation comprenant du salirasib selon la revendication 1, dans laquelle il comprend le sel de 1,2-distéaroyl-glycérol-3-phospho-éthanolamine-N [méthoxy (PEG) -2000] ammonium (DSPE-PEG) en tant que conjugué lipide-PEG.

5. Formulation comprenant du salirasib selon la revendication 1, dans laquelle la chaîne de PEG a un poids moléculaire de 2000 Da.

6. Formulation comprenant du salirasib selon la revendication 1, dans laquelle la quantité de FTA est comprise entre 1 %-30% de la quantité de polymère en poids (poids / poids).

7. Formulation comprenant du salirasib selon la revendication 1, dans laquelle la quantité de FTA est comprise entre 5%-20% de la quantité de polymère en poids (poids / poids).

8. Formulation comprenant du salirasib selon la revendication 1, dans laquelle la quantité de FTA est de 5% de la quantité de polymère en poids (poids / poids).

9. Formulation comprenant du salirasib selon la revendication 1, dans laquelle elle est obtenue par le procédé ci-dessous:
a) Phase organique (OP) contenant du PLGA est préparée,
b) FTA est ajouté à la phase organique,
c) Lécithine, DSPE-PEG et DOTAP sont dissous séparément dans de l'éthanol à 4% (v / v) préférentiellement, puis mélangés en quantité appropriée, une quantité appropriée d'eau désionisée est ajoutée à ce mélange,
d) La phase aqueuse (AP) est ajoutée à la phase organique préférentiellement dans un rapport de 10: 1,
e) Le mélange OP et AP est soniqué pendant 5 min en utilisant un sonicateur de bain à une fréquence de 42 kHz et une puissance de 100 W,
f) Les nanoparticules formées sont centrifugées dans des tubes filtrants-centrifuges contenant une membrane perméable de taille moléculaire de 10 kDa (Millipore),
g) Les nanoparticules sont lavées trois fois en utilisant du PBS (pH 7,4),
h) Les dispersions de nanoparticules sont congelées à -80°C et lyophilisées pendant 24 heures.

10. Formulation comprenant du salirasib selon la revendication 1, dans laquelle elle est administré par voie intraveineuse.

11. Formulation comprenant du salirasib selon la revendication 1, dans laquelle elle comprend l'administration intratumorale.

12. Formulation comprenant du salirasib selon la revendication 1 pour une utilisation dans le traitement de tumeurs cérébrales.
